# EUROPEAN PATENT SPECIFICATION

(11) **EP 2 176 411 B1**
(45) Date of publication and mention of the grant of the patent: **18.04.2012**
(21) Application number: 08773320.0
(22) Date of filing: 23.07.2008
(51) Int. Cl.: C12N 15/11, A61K 31/713, C12N 15/87

(54) **NANOPARTICLE-MEDIATED TREATMENT FOR INFLAMMATORY DISEASES**
NANOPARTIKEL-VERMITTELTE BEHANDLUNG FÜR ENTZÜNDUNGSKRANKHEITEN
TRAITEMENT INDUIT PAR NANOPARTICULE DESTINÉ AUX MALADIES INFLAMMATOIRES

(30) Priority: 23.07.2007 DK 200701079
(43) Date of publication of application: 21.04.2010
(73) Proprietor: Aarhus Universitet, 8000 Århus C (DK)
(72) Inventor: KJEMS, Jørgen, DK-8240 Risskov (DK); HOWARD, Kenneth Alan, DK-8200 Århus N (DK); BESENBACHER, Flemming, DK-8210 Århus V (DK); DELEURAN, Bent, DK-8230 Åbyhøj (DK)
(74) Representative: Plougmann & Vingtoft A/S
(86) International application number: PCT/DK2008/050184
(87) International publication number: WO 2009/012786

(56) References cited:
- WO-A-2009/006905
- KHOURY M. ET AL.: "Efficient new cationic liposome formulation for systemic delivery of small interfering RNA silencing Tumor Necrosis factor alpha in experimental arthritis" ARTHRITIS & RHEUMATISM, vol. 54, no. 6, June 2006 (2006-06), pages 1867-1877, XP002524666
- LIU X. ET AL.: "The influence of polymeric properties on chitosan/siRNA nanoparticle formulation and gene silencing" BIOMATERIALS, ELSEVIER SCIENCE PUBLISHERS BV., BARKING, GB, vol. 28, no. 6, 28 November 2006 (2006-11-28), pages 1280-1288, XP005782068 ISSN: 0142-9612
- TAN W.B. ET AL.: "Quantum-dot based nanoparticles for targeted silencing of HER2/neu gene via RNA interference" BIOMATERIALS, ELSEVIER SCIENCE PUBLISHERS BV., BARKING, GB, vol. 28, no. 8, March 2007 (2007-03), pages 1565-1571, XP005821955 ISSN: 0142-9612
- ROY K. ET AL.: "Oral gene delivery with Chitosan-DNA nanoparticles generates immunologic protection in a murine model of peanut allergy" NATURE MEDICINE, vol. 5, no. 4, April 1999 (1999-04), pages 387-391, XP002524667
- YEO Y. ET AL.: "Peritoneal application of chitosan and UV-cross-linkable chitosan" JOURNAL OF BIOMEDICAL MATERIALS RESEARCH, vol. 78A, no. 4, 15 September 2006 (2006-09-15), pages 668-675, XP002524668
- BEREZOVSKAYA O. ET AL.: "Colony Stimulating Factor-1 potentiates neuronal survival in cerebral cortex ischemic lesion" ACTA NEUROPATHOLOGICA, vol. 92, no. 5, 1996, pages 479-486, XP002525382

## Description

### Background

### Delivery of nucleic acids

Delivery of nucleic acids into target cells is of interest for various reasons. For example, the use of expression plasmids and antisense molecules as gene therapy drugs has been hampered by poor delivery techniques. Furthermore, an improved delivery technique could expand the use of so-called aptamers, to also include cellular targets. Aptamers are nucleic acids that fold into a three-dimensional structure that allow them to interact with target molecules at high affinity and specificity.

Another class of nucleic acids that has attracted massive attention recently is small interfering RNAs and micro RNAs. These double stranded RNA complexes can mediate various modifications of target nucleic acids in the cell. In this process, the antisense strand of the complex acts as a guide, as the antisense strand can hybridise to target nucleic acids that have stretches of sequence complementarily to the antisense strand.

RNA-mediated knockdown of protein expression at the messenger RNA (mRNA) level offers a new therapeutic strategy to overcome disease. The process by which small interfering RNA (siRNA) modulate enzymatic-induced cleavage of homologous mRNA and concomitant interruption of gene expression (RNA interference) has been extensively studied as a tool for investigating cellular processes in mammalian cells. RNA interference has been demonstrated using siRNA directed against genes responsible for viral pathogenesis, cancer and inflammatory conditions. As a consequence, the possibility to silence genes implicated in disease using siRNA has led to a rapidly evolving area in drug discovery.

RNA molecules may also induce transcriptional silencing. RNA-induced transcriptional silencing is a form of RNA interference by which short RNA molecules - microRNA (miRNA) or small interfering RNA (siRNA) - trigger the downregulation of transcription of a particular gene or genomic region. This is usually accomplished by modification of histones, often by methylation, or by the induction of heterochromatin formation.

The effectiveness of a drug is determined by the ability to migrate through the body and reach target sites at therapeutically relevant levels. Injection of naked siRNA into mice has resulted in localized gene knockdown. A drawback, however, is the requirement for high doses due to RNA instability and non-specific cellular uptake and impracticality of this method for human use. Viral and non-viral delivery systems have been developed in order to overcome extracellular and intracellular barriers that restrict therapeutic use of nucleic acid-based drugs.

Polyelectrolyte nanoparticles formed by self assembly of plasmid DNA with polycations, widely used in gene delivery, have been adopted for siRNA use. Cationic polymer and lipid-based siRNA vectors have been shown to enter cells and mediate specific RNA interference *in vitro* and *in vivo.* The effectiveness of nanoparticle systems is limited by rapid clearance from the circulation either by mononuclear phagocytic cell capture or non-specific cationic interactions with biological membranes and connective tissue. An alternative approach for both local and systemic drug delivery is exploitation of mucosal routes, e.g. nasal, to avoid the first pass hepatic clearance mechanism associated with intravenous administration. The polysaccharide chitosan, successfully used for nasal drug delivery due to mucoadhesive and mucosa permeation properties, has been utilised in the formation of polyelectrolyte nanoparticles containing plasmids for gene expression in respiratory sites and systemic tissue.

### Inflammatory disease

Inflammation involves a complex network of proinflammatory cytokine responses e.g. IL-2, IL-4, IL-7, IL-12, IFNs, GM-CSF or TNF-alpha mediated by immune cells such as macrophages, dendritic cells and T and B lymphocytes. The production of Tumour Necrosis Factor (TNFα) by macrophages plays a predominant role in the development and progression of rheumatoid arthritis, among others. Inflammatory responses usually involve systemic and local production of cytokines e.g systemic production of TNFα from circulatory macrophages and local production from infiltrating macrophages in the joints in the acute and chronic phase of rheumatoid arthritis

Rheumatoid arthritis (RA) affects around 1% of the population, mainly women in the 40' and is a destructive joint disease that untreated has decreased life expectance of 8 years.

Treatment today is methotrexate in combination with corticosteroids either injected into the joints or given systemically.

This treatment is effective in less that 50% of the patients and is often combined with side effects that make it impossible to give an effective treatment of the patient.

In case of treatment failure, anti-TNFα antibodies are used increasingly. Initially these usually work well, but after 2 years of treatment only 50% of the patients have a good clinical response. The treatment failure is mainly caused by formation of neutralizing anti TNFα antibodies or severe infections due to the systemic affect on the immune system by the antibodies. The treatment cost for anti-TNFα antibodies is around 20.000 euros pr patient year.

There is therefore still a great need for new treatments.

RNA interference targeting knockdown of proinflammatory production in macrophages offers a new approach. Studies using lipid-based delivery of anti-TNFα siRNA by the intravenous and local injection into the joints have previously been shown to be effective in reducing local inflammation. The rational is based on siRNA delivery into the joints for the site of siRNA knockdown.

The intravenous route, however, offers a number of extracellular barriers that restricts this approach including mononuclear phagocyte removal or breakdown of the delivery system in the bloodstream. Local injection requires specialized personnel for administration. These approaches combat the local site of inflammation but do not do not combat the systemic and chronic phase of the disease.

Khoury et al. (Arthritis & Rheumatism, vol. 54, no. 6, June 2006, pages 1867-77) discloses a cationic liposome formulation comprising siRNA targeting TNFα, and applications thereof in rheumatoid arthiritis.

Liu et al. (Biomaterials, vol. 28, no. 6, 28 November 2006, pages 1280-88) discloses a chitosan/siRNA nanoparticle formulation as a delivery system for gene silencing. Nanoparticles having a molecular weight of more than 10 kDa are discloses.

WO 2009/006905 discloses chitosan/siRNA nanoparticles comprising a siRNA targeting an mRNA encoding a pro-inflammatory cytokine. There is no mentioning of intraperitoneal delivery.

Tan et al. (Biomaterials, vol. 28, no. 8, March 2007, pages 1565-71) discloses a chitosan/siRNA nanoparticle formulation as a delivery system for gene silencing. Nanoparticles having a molecular weight of more than 10 kDa are discloses.

Roy et al. (Nature Medicine, vol. 5, no. 4, April 1999, Pages 387-91) discloses efficient gene delivery of chitosan-DNA particles through oral administration, for mucosal DNA vaccination.

Yeo et al. (Journal Biomed. Mat. Res., bol. 78A, no. 4, 15 September 2006, pages 668-75) discloses peritoneal application of chitosan as a drug delivery system highlighting some negative effects relating to this route of administration.

Berezovskaya et al. (Acta Neuropathologica, vol. 92, no. 5, 1996, pages 479-86) discloses the delivery of CSF-1 in chitosan microcapsules implanted intraperitoneally.

### Summary of the invention

The present disclosure provides nanoparticles comprising chitosan and a siRNA targeting an mRNA encoding a pro-inflammatory cytokine, such as e.g. TNF-alpha. The nanoparticles may be used for treatment, preferably treatment of an inflammatory disease.

### Brief description of the drawings

Fig. 1
   In vitro TNFα knockdown in peritoneal macrophages, see example 1 for details.
Fig. 2
   In vivo TNFα knockdown in peritoneal macrophages, see example 2 for details.
Fig. 3
   Inflammation arrestment induced by I.P administration of chitosan/siRNA nanoparticles. See example 3 for details.
Fig. 4
   Histological evaluation of joints from example 3.
Fig. 5
   Prophylactic inhibition of arthritis. See example 5 for details.
Fig. 6
   Northern Blot analysis for chitosan/siRNA complexes in the blood and organs of mice after I.P. injection. See example 6 for details.

In a first aspect, the present invention relates to the use of a chitosan/siRNA nanoparticle comprising a siRNA that targets an mRNA encoding a pro-inflammatory cytokine for the preparation of a medicament for intraperitoneal delivery. In an embodiment of the present invention is the pro-inflammatory cytokine selected from the group consisting of IL-1, IL-2, IL-4, IL-7, IL-12, INFs, GM-CSF or TNF-alpha.

A second aspect of the present invention relates to the use of a chitosan/sIRNA, wherein the chitosan has a molecular weight of more than 10 kDa, for the preparation of a medicament for intraperitoneal delivery.

### Disclosure of the invention

The peritoneum contains a reservoir of macrophages that are implicated in the acute and chronic phases of inflammatory diseases. The use of a nanoparticle system containing anti-TNFα agent that targets this cell population would enable treatment of both systemic and acute phases. The intraperitoneal route does not require the nanoparticle to migrate in the bloodstream so nanoparticle integrity is not compromised. The particulate nature of chitosan nanoparticle and macrophage-rich environment in the peritoneum affords avid uptake into phagocytic cells such as macrophages. In this approach a simple passive targeting strategy for knockdown of TNFα in cells implicated in acute and chronic inflammation can be used. The requirement, however, is the ability of a nanoparticle system to knockdown TNFα production in "difficult-to-gene silence" macrophage cells.

As documented in the examples section, the inventors have surprisingly been able to deliver the siRNA of a chitosan/siRNA nanoparticle to peritoneal macrophages and downregulate the target mRNA of the siRNA. siRNAs can be directed to most, if not all, mRNAs by way of complementarity between the guide strand of the siRNA and the target mRNA.

Thus, the term siRNA as used herein is a RNA complex that recruits the so-called RNA-Induced-Silencing-Complex (RISC) and mediates translational repression or degradation of target mRNAs. Preferred is degradation of target mRNAs. The skilled artisan will recognize that different siRNA structures exist and that they may be used interchangeably. Typically, the siRNAs are double stranded RNAs of 20-23 nt with 3'overhangs. However, the siRNAs may be longer, e.g. 27 nt or longer. Such longer siRNAs have been referred to as dicer substrates. The siRNAs may also be blunt ended, have 5'overhangs and they may be chemically modified, e.g. with LNAs or 2'O-methyls.

As an alternative to siRNAs, also RNase H inducing antisense oligonucleotides may be used as these can be targeted to the same mRNAs and mediate degradation. Thus, in one embodiment, the nanoparticle comprises a siRNA or an RNase H inducing antisense oligonucleotides and it should be clear that whenever reference is to a siRNA, a RNase H inducing antisense oligonucleotides may be used instead.

However, siRNAs are preferred over RNase H inducing antisense oligonucleotides.

Since the nanoparticles targets the cells involved in the inflammatory response, a low dose can be given. Preferably, the dose is selected from the group consisting of less than 50 mg/kg/day, less than 40 mg/kg/day, less than 30 mg/kg/day, less than 20 mg/kg/day, less than 10 mg/kg/day, less than 5 mg/kg/day and less than 1 mg/kg/day.

In another preferred embodiment, the dose is selected from the group consisting of less than 50 mg/kg/week, less than 40 mg/kg/week, less than 30 mg/kg/week, less than 20 mg/kg/week, less than 10 mg/kg/week, less than 5 mg/kg/week and less than 1 mg/kg/week.

When referring to dose, reference is to the amount of siRNA in the dose. In another embodiment, reference is to the amount of nanoparticle in the dose.

Thus, in a first aspect of the present invention provides the use of a chitosan/siRNA nanoparticle comprising a siRNA that targets an mRNA encoding a pro-inflammatory cytokine, preferably selected from the group consisting of IL-1, IL-2, IL-4, IL-7, IL-12, IFNs, GM-CSF or TNF-alpha, for the preparation of a medicament for intraperitoneal delivery.

Preferably, the cytokine is of mammalian origin. Even more preferably, they are of human origin. In another embodiment, the cytokine is of animal origin. As is known to the skilled man, siRNAs can be targeted to mRNA by way of complementary between the guide strand of the siRNA and the mRNA.

In a preferred embodiment, the nanoparticle of the invention is used as a medicament.

In one embodiment, the nanoparticle of the disclosure is used for treatment of an inflammatory disease, preferably selected from the group consisting of rheumatoid arthritis, Crohn's disease, multiple sclerosis, and psoriasis.

In another embodiment, the inflammatory disease is selected from the group consisting of inflammatory bowel disease (IBD) including ulcerative colitis and Crohn's disease; surgical adhesions; periodontal disease; polycystic kidney disease; chronic inflammatory diseases of the respiratory tract including asthma, chronic obstructive pulmonary disease (COPD), chronic bronchitis, asthmatic bronchitis, chronic obstructive bronchitis, and emphysema and other diseases which lead to chronic airway obstruction; diseases associated with the obstruction of body passageways, including for example, vascular diseases, neoplastic obstructions, inflammatory diseases, and infectious diseases; and, neovascular diseases of the eye including for example, corneal neovascularization, neovascular glaucoma, proliferative diabetic retinopathy, retrolental fibroblasia and macular degeneration.

Preferably, the subject to be treated for inflammatory disease is a mammal. Even more preferably the subject is a human. In another embodiment, the subject is a non-human mammal.

The nanoparticle of the invention is for intraperitoneal delivery (administration), preferably for treatment of an inflammatory disease.

In another embodiment, the nanoparticle is for co-administration with an immunosuppressive compound selected from the group of glucocorticoids, cytostatics, antibodies, drugs acting on immunophilins, other drugs such as interferons, opioids, TNF binding proteins and mycophenolate.

### Preparation of nanoparticle

Preferably, the siRNA of the chitosan/siRNA nanoparticle comprise a sequence that targets an mRNA encoding a pro-inflammatory cytokine, preferably selected from the group consisting of IL-1, IL-2, IL-4, IL-7, IL-12, IFNs, GM-CSF or TNF-alpha.

Preferably, the nanoparticle of the disclosure is prepared by a method comprising
a. Providing a chitosan solution
b. Providing an siRNA solution
c. Mixing the solution of step a with the solution of step b
d. Incubating the solution of step c under conditions of complex formation such that chitosan/siRNA nanoparticles form.

In one embodiment, the chitosan/siRNA nanoparticle comprises an initial crosslinker, such as polyphosphate.

In another preferred embodiment of the method of preparing a chitosan/RNA nanoparticle, the chitosan does not comprise an initial crosslinker.

The term initial cross linker is used to for a crosslinker that is added to chitosan to form a particle, before the RNA molecule is added. Typically in the art, e.g. when chitosan/plasmid nanoparticles are formed, the particles are preformed using an initial crosslinker such as polyphosphate. Thus, it is believed that the structure and activity of a particle formed without an initial crosslinker differ from that of a particle formed with an initial crosslinker. In particular, the use of an initial crosslinker seems to imply that the RNA will be distributed at the surface of the preformed particle, whereas when using the RNA as crosslinker, the RNA will be distributed evenly through the particle. An even distribution is expected to a positive effect on the biostability of the RNA molecules of the nanoparticle, as they will be less accessible for RNases.

Moreover, the omission of the initial crosslinker provides are more facile method of preparation. Instead of a two-step method, where the particles are formed first and then the RNA is added, a one-step method is provided in which the RNA and chitosan is mixed to form nanoparticles directly.

Thus, in a preferred embodiment, the RNA functions as a crosslinker in the formation of a nanoparticle. In other words, the RNA is the formactive component.

### Concentrations

### RNA concentration

In one of embodiment of the method of preparing a chitosan/RNA nanoparticle, the RNA solution comprises RNA at a concentration selected from the group consisting of at least 5 µM, at least 10 µM, at least 20 µM, at least 30 µM, at least 40 µM, at least 50 µM, at least 60 µM, at least 70 µM, at least 80 µM, at least 90 µM and at least 100 µM.

### Chitosan concentration

In another embodiment, the chitosan solution comprises chitosan at a concentration from the group consisting of at least 50 µg/ml, at least 60 µg/ml, at least 70 µg/ml, at least 80 µg/ml, at least 90 µg/ml, at least 100 µg/ml, at least 110 µg/ml, at least 120 µg/ml, at least 130 µg/ml, at least 140 µg/ml, at least 150 µg/ml, at least 160 µg/ml, at least 170 µg/ml, at least 180 µg/ml, at least 190 µg/ml, at least 200 µg/ml, at least 250 µg/ml, at least 500 µg/ml, at least 750 µg/ml and at least 1000 µg/ml.

### Degree of deacetylation

Preferably, the chitosan has a relatively high degree of deacetylation. Thus, in one embodiment, the chitosan has a degree of deacetylation of selected from the group consisting of at least 60%, least 65%, least 70%, least 75%, least 80%, least 85% and at least 95%.

### Molecular weight of chitosan

The molecular weight of the chitosan is preferably more than 10 kDa. In another embodiment, the molecular weight is more than 50 kDa and even more preferred is a molecular weight of more than 100 kDa.

Chitosan samples with a molecular weight in the range of 100-170 kDa and a deacetylation degree are particular favourable.

### N:P ratio

The above mentioned parameters can all be used to control the characteristics of the formed nanoparticle. Another important parameter is the so-called N:P ratio, defined herein as the ratio of chitosan amino groups (N) to RNA phosphate groups (P).

In a preferred embodiment, the nanoparticle is formed at a N:P ratio larger than 25. Experiments document that increasing the N:P ratio, leads to larger particles. In other embodiments, the N: P ratio is selected from the group consisting of a N: P ratio larger than 60, larger than 70, larger than 80, larger than 90, larger than 100 and larger than 150.

In this preferred embodiment, wherein the N:P ratio is larger than 70, the RNA solution comprises RNA at a concentration lower than 100 µM, such as lower than 90 µM, lower than 80 µM, lower than 70 µM, lower than 60 µM, lower than 50 µM, lower than 40 µM, lower than 30 µM, lower than 20 µM, lower than 10 µM, lower than 5 µM or lower than 1 µM.

When employing a high N:P ratio and a low RNA concentration, nanoparticles can be formed that comprises loosely bound chitosan.

As the degree of loosely bound chitosan is dependent on both the concentration of RNA in the RNA solution and on the N:P ratio, the skilled worker will appreciate how to manipulate these parameters to create nanoparticles with loosely bound chitosan. E.g. a high RNA concentration of the RNA solution may be used, if also the N:P ratio is kept high, i.e. a high concentration of chitosan is used.

In one embodiment, the nanoparticle comprising loosely bound chitosan has a high N:P ratio.

A nanoparticle with loosely bound chitosan is of interest e.g. to improve mucosal delivery. Therefore, in one embodiment, the nanoparticle with loosely bound chitosan is for mucosal delivery.

A nanoparticle particle with a discrete character is of interest e.g. for systemic delivery. Such a particle can also be formed by controlling various parameters involved in the method of forming the nanoparticle. Particularly, a low N:P ratio favours the formation of a discrete nanoparticle. As mentioned above, the concentration of RNA and chitosan can be varied while maintaining a reasonably constant N:P ratio.

### Concentrated Method vs. N:P ratio

In this embodiment, the N:P ratio is lower than 70 such as but not limited to a N:P ratio lower than 60, lower than 50, lower than 40, lower than 30, lower than 20 or lower than 10, respectively.

In one embodiment, the nanoparticle of discrete character has a low N:P ratio. In another embodiment, the concentration of the RNA solution is at least 100 µM, such as but no limited to at least 250 µM, at least 200 µM, at least 150 µM, at least 90 µM, at least 80 µM, at least 70 µM, at least 60 µM or at least 50 µM, respectively.

Using a high concentration of RNA in the RNA solution turns out to have several advantages. As outlined in the examples section, when the particles are formed using a RNA solution with a concentration of 250 µM, the particles are more discrete and monodispersed, as compared to particles formed using a pre-diluted RNA solution of 20 µM (as can be seen from the PDI measurements in table 3). Moreover, it surprisingly turns out that the nanoparticles formed using the concentrated RNA solution have a more specific effect, i.e. they do not give rise to any non-specific knockdown, which may be the case for particles formed using a RNA solution with a lower concentration of RNA.

Furthermore, using a high concentration of RNA in the RNA solution allows particle formation at a low pH such as ph 4.5, which in turn makes the particles more stable. Using a slightly higher pH of 5.5 is also possible. A pH of 5.5 may decrease detrimental effects of acetate buffer on cell viability.

Additionally, using a high concentration of RNA in the RNA solution means that the amount of RNA in particles increase, which decreases the amount of particle solution that has to be administered to a cell or organism.

In another embodiment, the chitosan concentration is less than 250 µg/ml.

In a particular preferred embodiment, the chitosan concentration is less than 250 µg/ml, while the RNA concentration is higher than 100 µM.

By controlling the concentrations of RNA and chitosan, and thereby the N:P ratio, also the size of the particles can be controlled, (as documented in the examples section. Thus, in one embodiment, the size of the particle is between 10 and 200 nM.

In another embodiment, the formed particles are discrete in form and have a polydispersity index lower than 0,4.

### Use of the nanoparticle

One aspect of the disclosure is the use of the nanoparticle of the invention for the preparation of a medicament for the treatment of an inflammatory disease.

Preferably, the subject to be treated for inflammatory disease is a mammal. Even more preferably the subject is a human. In another embodiment, the subject is a non-human mammal.

Other inflammatory diseases include inflammatory bowel disease (IBD) including ulcerative colitis and Crohn's disease; surgical adhesions; periodontal disease; polycystic kidney disease; chronic inflammatory diseases of the respiratory tract including asthma, chronic obstructive pulmonary disease (COPD), chronic bronchitis, asthmatic bronchitis, chronic obstructive bronchitis, and emphysema and other diseases which lead to chronic airway obstruction; diseases associated with the obstruction of body passageways, including for example, vascular diseases, neoplastic obstructions, inflammatory diseases, and infectious diseases; and, neovascular diseases of the eye including for example, corneal neovascularization, neovascular glaucoma, proliferative diabetic retinopathy, retrolental fibroblasia and macular degeneration.

In a preferred embodiment, the medicament is for co-administration with an immunosuppressive compound selected from the group of glucocorticoids, cytostatics, antibodies, drugs acting on immunophilins, other drugs such as interferons, opioids, TNF binding proteins and mycophenolate.

Preferably, the inflammatory disease is selected from the group consisting of rheumatoid arthritis, Crohn's disease,multiple sclerosis and psoriasis.

One aspect of the invention is use of the nanoparticle of the invention for preparation of a medicament for intraperitoneal delivery.

One aspect of the disclosure is a pharmaceutical composition comprising the nanoparticle of the invention.

In a preferred embodiment, the pharmaceutical composition further comprises an immunosuppressive compound selected from the group of glucocorticoids, cytostatics, antibodies, oligonucleotide based drugs such as siRNAs, microRNAs, microRNA inhibitors and RNase H inducing antisense molecules, drugs acting on immunophilins, other drugs such as interferons, opioids, TNF binding proteins and mycophenolate.

In a preferred embodiment, co-administration of the nanoparticle with immunosuppressive compound lowers the amount of immunosuppressive compound that has to be administrated. I.e. the pharmaceutical composition comprises the immunosuppressive compound at a reduced dose as compared to administration without the nanoparticle of the invention.

One aspect of the disclosure is a method of delivering an effective amount of the nanoparticle of the invention to a person in need thereof, said method comprising an intraperitoneal injection of a pharmaceutical composition comprising the nanoparticle.

Preferably, the nanoparticle targets macrophages.

Preferably, the person in need has an inflammatory disease.

More preferably, the person in need has an inflammatory disease selected from the group consisting of rheumatoid arthritis, Crohn's disease, multiple sclerosis and psoriasis.

### Examples

The examples describe chitosan/siRNA nanoparticle-mediated TNF-α knockdown in peritoneal macrophages as a method to reduce inflammation at local and systemic sites presenting a novel treatment strategy for inflammatory disease.

### MATERIALS AND METHODS

siRNA sequences TNF-α Dicer substrate (DsiRNAs) [1] containing the sequence: sense, 5'-GUCUCAGCCUCUUCUCAUUCCUGCT-3', antisense 3'-AGCAGGAAUGAGAAGGGCUGAGACAU-5'; DsiRNAs negative control containing the sequence: sense, 5'CUUCCUCUCUUUCUCUCCCUUGUGA-3', antisense 3'-UCACAAGGGAGAGAAAGAGAGGAAGGA-5' EGFP siRNA containing a fluorescent Cy3 labelled 5'sense strand was used for cellular uptake studies 5'Cy3-T*CCUUCCUCUCUUUCUCUCCCUUGUG*A-3', antisense 5'-Cy3-T*CACAAGGGAGAGAAAGAGAGGAAGG*A

### Formation of chitosan/siRNA nanoparticles.

Chitosan was dissolved in sodium acetate buffer (0.2M NaAc, pH 4.5) to obtain a 1 mg/ml solution and then adjusted to pH 5.5. 20 µl of siRNA (100 µm) in nuclease free water was added to 1 ml of filtered chitosan (800 µg/ml) whilst stirring and left for 1 h. To calculate specific N: P ratios (defined as the molar ratio of chitosan amino groups/RNA phosphate groups) a mass-per-phosphate of 325 Da was used for RNA and mass-per-charge of chitosan 167.88 (84% deacetylation). Nanoparticles formed at N:P were used in all experiments.

### Photon correlation spectroscopy and determination of surface charge on chitosan/siRNA nanoparticles.

The hydrodynamic size of chitosan/siRNA nanoparticles were determined by photon correlation spectroscopy (PCS) using a Zetasizer Nano ZS. PCS was performed at 25°C in sodium acetate buffer in triplicate with sampling time and analysis set to automatic. Particle size is presented as the z-average of three measurements +/- SD. Surface charge was measured by determination of zeta potential using a Zetasizer Nano ZS in sodium acetate buffer.

### TNFα knockdown in murine peritoneal macrophages in vitro.

Six-eight-week-old female C57BL/6J mice were killed by cervical dislocation and injected intraperitoneally with 5 ml of wash medium (PBS supplemented with 5% LPS-free heat-inactivated fetal calf serum (FCS) (Cambrex, Baltimore, MD, USA) and 4 µl/ml heparin. The abdomen was agitated gently, the peritoneum exposed and breached, and the media removed using a syringe. The suspension was centrifuged (450 x g for 10 min) and the pellet resuspended in RPMI 1640 supplemented with 10 % FCS and antibiotics. The suspension was plated on a 96-well cell culture plate at 350.000 cells per well in 100 µl. The macrophages were allowed to adhere for 2 h before media containing non-adherent cells was removed. Fresh pre-heated media was then added the cells. After 20 h the media was removed and replaced with serum-free RPMI1640 and chitosan/siRNA nanoparticles added at 50 mM siRNA concentration. After 4 h, media was removed and replaced with fresh media containing 10 % FCS. After 24 or 48 h the cells were stimulated with LPS (100 ng/ml). Supernatants were harvested 5 h later and TNFα was detected by an enzyme-linked immunosorbent assay (ELISA). Maxisorp plates were coated overnight at room temperature with 100 µl of goat anti-mouse TNF-α capture antibody at a concentration of 2 µg/ml in coating buffer (15 mM Na2CO3, 35 mM NaHCO3, 0.02 % NaN3 [pH 9.6]). After blocking for at least 1 h at room temperature with 300 ul of 1% bovine serum albumin (BSA) in blocking buffer (phosphate-buffered saline with 5 % sucrose and 0.05 % NaN3 [pH 7.4]), successive culture supernatants or recombinant murine TNF-α was added to the wells (100 µl each) and incubated overnight at 4°C. Subsequently, wells were incubated at room temperature for 2 h with 100 µl biotinylated, goat anti-mouse TNFα detection antibody (150 ng/ml). Streptavidin-horseradish peroxidase diluted at 1:200 in TBS with 0.1% BSA was added, and the mixture incubated for 20 min. H2O2 and tetramethylbenzidine was added and plates were incubated in the dark for an appropriate amount of time to allow colour development. The colour reaction was stopped with 50 µl 5% H2SO4 and the absorbance measured at 450 nm with 570 nm as a reference. After each step, the plates were washed three times with PBS 0.05% Tween 20, pH 7.4.

Transfection and TNF-a knockdown in murine peritoneal macrophages in vivo. 200µl of chitosan/siRNA nanoparticles (Dicer substrate TNF-α and negative controls or Cy3-labelled siRNA) were injected intraperitoneally into C57BL/6J mice. Peritoneal macrophages were isolated, according to the procedure described above, 2 or 24 h post-injection and plated in 96-well plates or mounted with DAPI nuclear stain onto glass slides. Uptake of Cy3-labelled siRNA was monitored by a Zeiss semi-confocal epifluorescence microscope. TNF-α levels in the harvested supernatants of LPS stimulated macrophages were measured 2 h and 24 h post-injection according to the above protocol.

### Inflammatory downregulation in collagen type II induced arthritic mice after i.p. administration of chitosan/siRNA nanoparticles.

The collagen type II DBA/I arthritic model was used to evaluate the antiinflammatory potential of chitosan/siRNA formulations. 40 six to nine week DBA/I mice were immunised subcutaneously on study day 0 with 0.1 ml of Anthrogen-CIA collagen emulsion prepared according to the manufacturer's protocol. The limbs of the mice were scored for clinical symptoms of arthritis under veterinary supervision at Pipeline Biotech A/S (Trige, Denmark). Between days 28 and 35, mice arthritic scores 1 to 3, were divided into 5 groups of 5. Mice were i.p. dosed with 200 µl of chitosan/siRNA nanoparticles (Dicer substrate and negative controls) on study day 29, 31, 33, 35 and 37. The abdomen was massaged gently after each administration to ensure uniform distribution throughout the peritoneal cavity. Mice administered subcutaneously on days 36 to 44 with Dexamethasone were used as a positive control. Animals were assessed for clinical symptoms from day 21 to day 41 using a standard scoring system for evaluation of joint inflammation (0 = normal, 1 = mild, 2 = moderate and 3 = severe, 4 = maximal; sum total for all 4 limbs 0-16). Mice were terminated on day 42 except those showing severe signs of inflammation which were sacrificied earlier. Paws were then fixed in formalin, decalcified, paraffin embedded and 2 um sections assessed for evidence of joint destruction and cellular inflammation. In addition, 100 µl of blood was drawn from the orbital plexus before immunisation and throughout the study to evaluate systemic cytokine levels.

### Measurement of systemic cytokine levels.

Expression of cytokines was measured using the Luminex Technology TM, and a 9-plex human cytokine kit purchased from Bio-Rad. Briefly, the filter plates were washed with assay buffer and 50 µl of freshly vortexed antibody-conjugated beads were added to each well. The plates were washed with assay buffer and serum samples and standards were added. After gently shaking (30 sec at 1.100 rpm), the plates were incubated at room temperature in the dark for 45 min under gentle agitation (300 rpm). After one wash, 25 µl of the detection antibody was added to each well, and the plates were agitated and incubated as above. Subsequently, the plates were washed and incubated for 10 min with 50 µl of a streptavidine-PE solution whilst shaking (30 sec at 1.100 rpm, 10 min 300 rpm). Finally the plates were washed and 125 µl of assay buffer was added to each well followed by shaking for 10 sec at 1100 rpm and immediate reading on the Bio-Plex Protein Array Reader.

### Example 1

This example shows the ability of the chitosan/siRNA nanoparticle system to knockdown the pro-inflammatory cytokine in "difficult to transfect cells" which has previously been a barrier to strategies targeting expresion in macrophages A) In vitro specific knockdown of TNFα expression in lipopolysaccarhide (LPS) stimulated (measured 4 h later) isolated mouse peritoneal macrophages 24 h and 48 h post-incubation (4 h) with nanoparticles containing TNF-α siRNA. A siRNA dose dependent knockdown shows that 50nM is better than 100nM but 10nM is to low. Interestingly, nanoparticles with low siRNA (10nM) seem to induce TNF-α expresion which could be due to immunostimulatory effect reported for chitosan. This is circumvented by use of higher TNFα siRNA. The knockdown effect is observed 24 and 48 h after nanoparticle addition. B) Specific knockdown of TNF-α with optimised siRNA concentration (50nM) in isolated mouse peritoneal macrophages stimulated with LPS 24h and 48 h post-incubation (4 h) with nanoparticles.

### Example 2

This example shows uptake and knockdown of TNF-α within the peritoneal macrophages in situ after intraperitoneal (i.p.) administration of chitosan/siRNA nanoparticles. A) Nanoparticles containing Cy5 labelled siRNA were administered i.p. to C57 mice and left for 2 h before macrophages were harvested, labelled with a macrophage marker and uptake visualised. Nanoparticles containing TNF-α were administered i.p. and left either 2 h (B) or 24 h (C) before peritoneal macrophages were harvested, stimulated with LPS and TNF-α levels measured. The histograms show specific knockdown (~ 40 %) with the TNF-α formulations. Inserts show some induction of IFN with the nanoparticle formulations.

### Example 3

This example shows arrested inflammation (measured by joint size score) in Type II collagen induced DBA/1J arthritic mice (n=5) after intraperitoneal administration of the chitosan/siRNA-TNF-a nanoparticles. It shows systemic i.p. administered nanoparticles have a local effect
A) Arthritic scores in mice i.p. administered with formulations on day 1, 3, 5, 7 and 9 (~5 µg siRNA per dose started after arthritis induction).
   In comparison with the negative control buffer and chitosan/siRNA mismatch nanoparticles, the chitosan/siRNA-TNF-α nanoparticles show lower arthritic scores. The dexamethasone positive control was administered daily subcutaneously.
   (Scoring 0 Normal; 1, Mild, but redness and swelling of joints; 2, Moderate redness and swelling; 3, Severe redness and swelling of the entire paw; 4, maximal inflammed limb)
B) Survival graph showing 100% survival with chitosan/siRNA-TNF-α nanoparticles, buffer and dexamethasone.

### Example 4

This example shows histological evaluation of joints taken from example 3.
A) The joints from dexamethasone and chitosan/siRNA-TNFα nanoparticles treated animals show intact cartilage with no signs of inflammatory cell invasion whilst the buffer and chitosan/siRNA mismatch nanoparticles show signs of cartilage destruction and cellular infiltration
B) Histological scoring of joints animals (n=5)

### Example 5

This example shows the treatment as a prophylactic by inhibition of arthritis in mice (n=8) after intraperitoneal administration of the chitosan/siRNA-TNF-α nanoparticles before collagen is administered. Delayed onset of inflammation measured by arthritic score can be seen in animals given chitosan/siRNA-TNF-α nanoparticles but not mismatch or untreated animals (animals dosed with nanoparticles on day -2, 1, 5, 9, 13, 17 and 21)

### Example 6

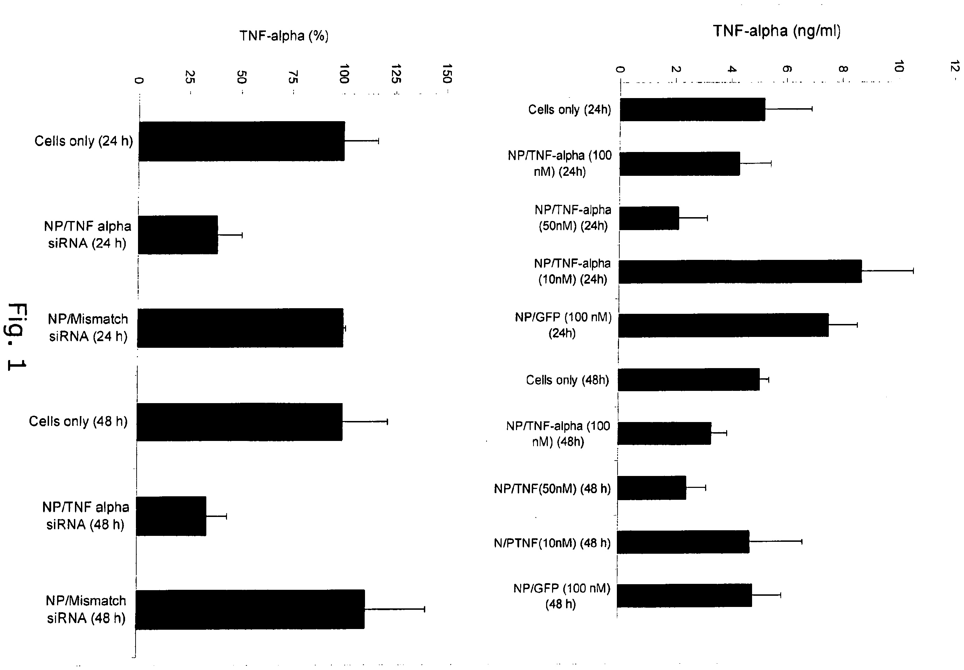

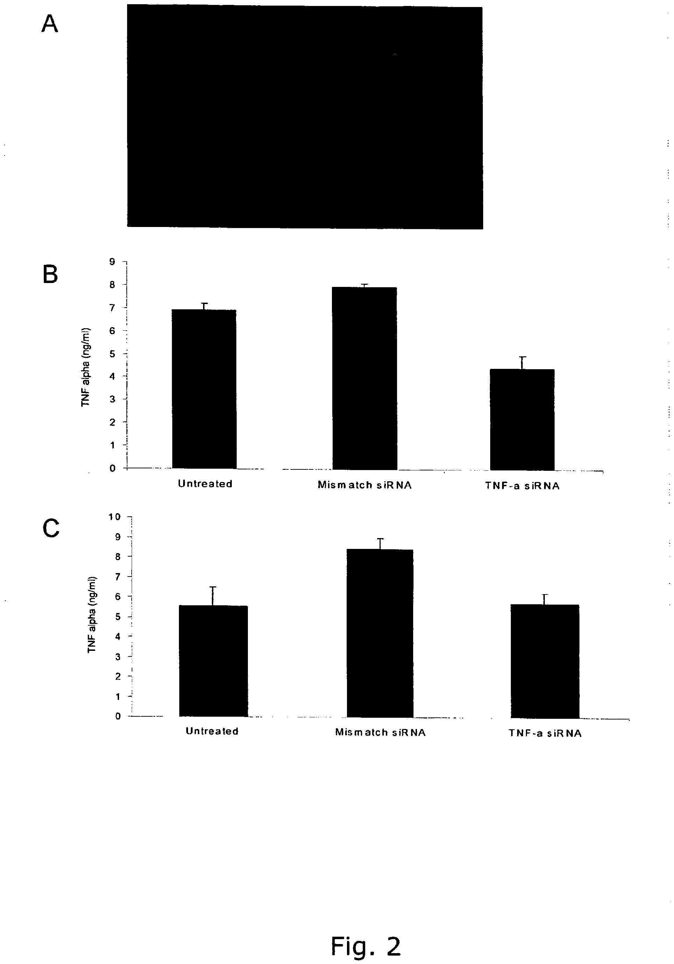

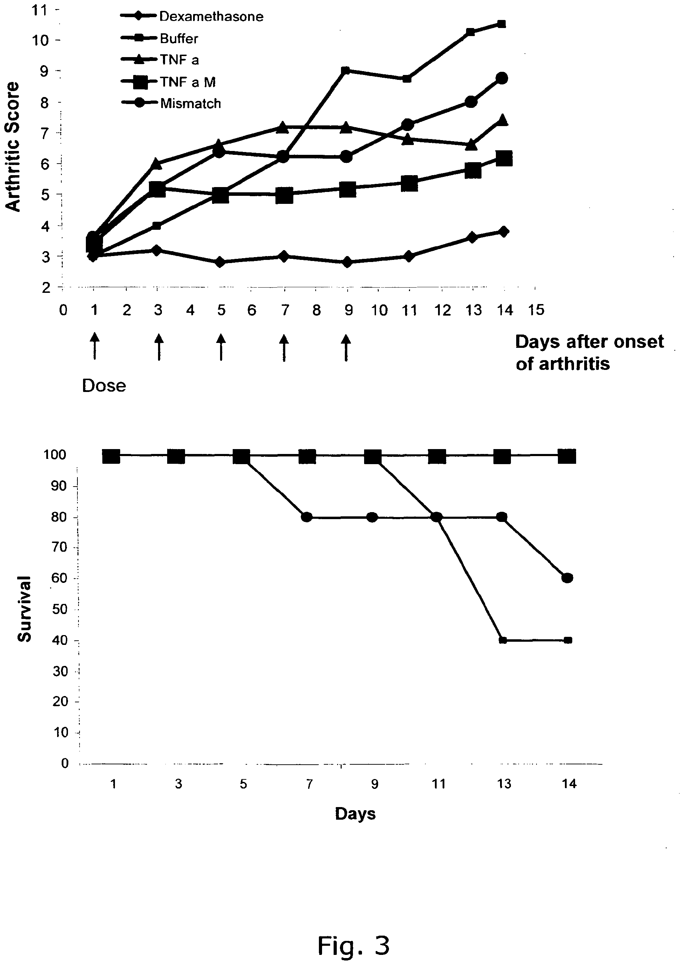

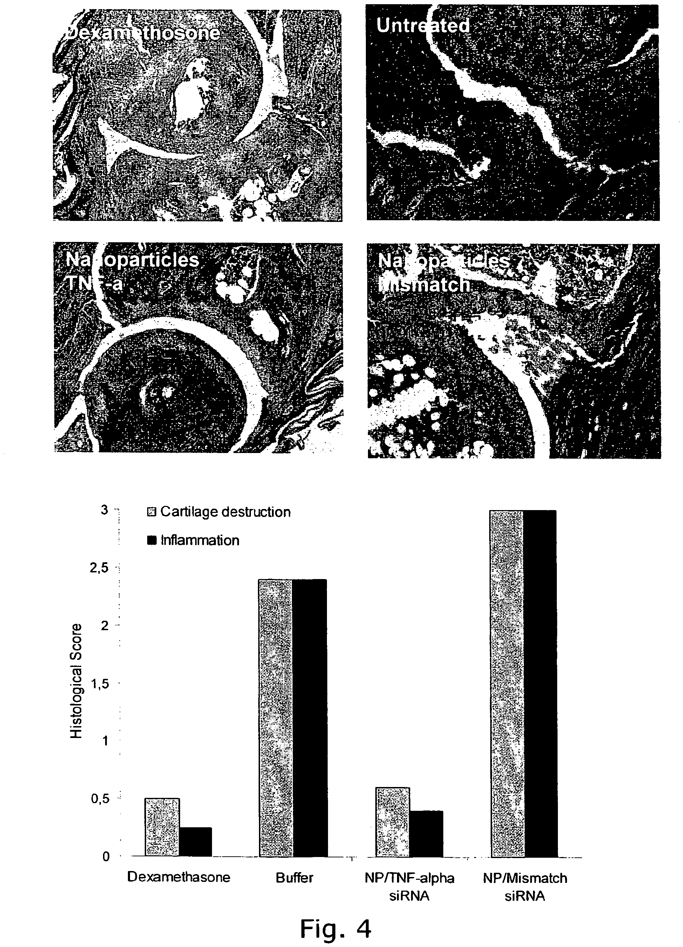

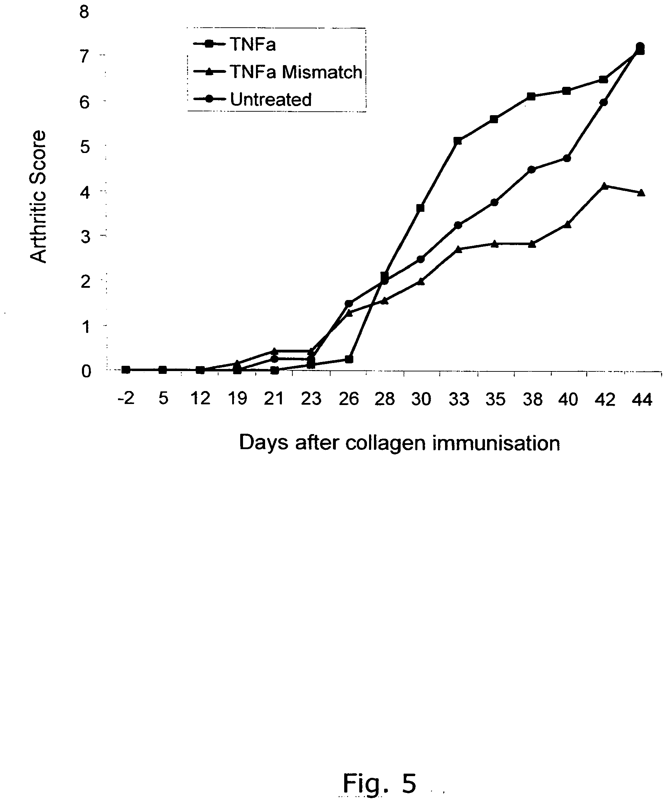

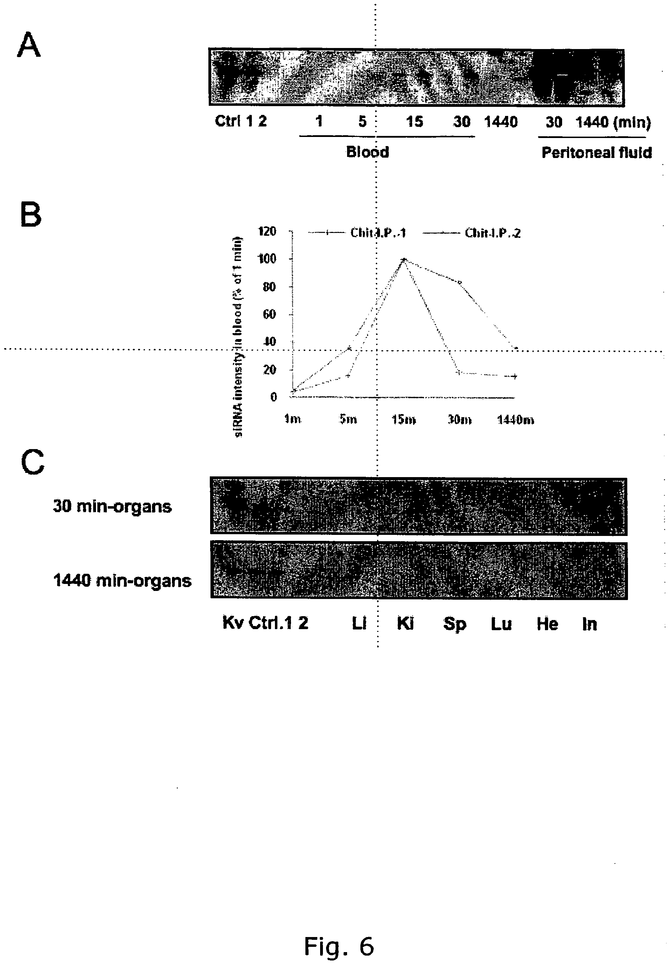

Northern Blot analysis for chitosan/siRNA complexes in the blood and organs of mice after I.P. injection:
Chitosan/siRNA complex was injected intraperitoneally (I.P.), containing 10 µg LNA modified siRNA in 200µl volume. Tissues and blood samples were collected at indicated time points and total RNA purified. Two µg RNA from blood and four µg RNA from tissues were run on 15% denaturing polyacrylamide gels, and Northern Blot was performed by probing with [γ-32P] ATP labeled sense strand LNA ending modified siRNA. The results are shown in figure 6:
   (A), RNA integrity is analysed for blood samples at indicated time course and peritoneal fluid at 30 minutes and 24 hours. Loading order is: lane 1, siRNA duplex, 2, injected chitosan/siRNA complex (represented as Ctrl.1 2), 3, blank, 4-13, as 1, 5, 15, 30 minutes and 24 hours blood, 14-17, as peritoneal fluid at 30 minutes and 24 hours from mouse 1 and 2, respectively.
   (B), The intensity of the siRNA signals from blood samples is measured by Quantity ONE programme. Percentages of the intensity at 15 minute are presented and clearance curves are made.
      (C) RNA integrity is analysed for tissue samples from six organs at 30 minutes and 24 hours. Loading order is: lane 1, one kidney sample from I.V. injection (Kv), 2, siRNA duplex, 3, injected chitosan/siRNA complex (represented as Ctrl.1 2), 4, blank, 5-16, as Liver (Li), Kidney (Ki), Spleen(Sp), Lung (Lu), Heart (He) and Small Intestines(In) from mouse 1 and 2 at 30 minute and 24 hour, respectively.

### Result and conclusions:

siRNA is still present in the peritoneal liquid after 30 mins. siRNA is clearly detected in the blood after 5 mins and peaks at 15 mins. It is detected in Kidney and lungs after 30 mins but not detectable after 24 hours.

The data shows a good majority of the particles retained in the peritoneum after 30 min which supports the delivery of nanoparticles into macrophages within this site

At all stages the siRNA appears to be full length (no partial degradation products) suggesting that chitosan protects the RNA towards degradation.

## Claims

1. Use of a chitosan/siRNA nanoparticle comprising a siRNA that targets an mRNA encoding a pro-inflammatory cytokine for the preparation of a medicament for intraperitoneal delivery.

2. The use according to claim 1, wherein the pro-infilammatory cytokine is selected from the group consisting of IL-1, IL-2, IL-4, IL-7, IL-12, IFNs, GM-CSF or TNF-alpha.

3. Use of a chitosan/siRNA nanoparticle, wherein the chitosan has a molecular weight of more than 10 kDa, for the preparation of a medicament for intraperitoneal delivery.

## Patentansprüche

1. Verwendung eines Chitosan-/siRNA-Nanopartikels, umfassend eine siRNA, deren Ziel eine ein proinflammatorisches Zytokin codierende mRNA ist, zur Herstellung eines Arzneimittels für die intraperitoneale Abgabe.

2. Verwendung nach Anspruch 1, wobei das proinflammatorische Zytokin ausgewählt ist aus der Gruppe, bestehend aus IL-1, IL-2, IL-4, IL-7, IL-12, IFNs, GM-CSF oder TNF-alpha.

3. Verwendung eines Chitosan-/siRNA-Nanopartikels, wobei das Chitosan eine Molekülmasse von mehr als 10 kDa aufweist, zur Herstellung eines Arzneimittels für die intraperitoneale Abgabe.

## Revendications

1. Utilisation d'une nanoparticule de chitosane/ARNsi comprenant un ARNsi qui cible un ARNm codant pour une cytokine pro-inflammatoire pour la préparation d'un médicament destiné à être administré par voie intrapéritonéale.

2. L'utilisation selon la revendication 1, dans laquelle la cytokine pro-inflammatoire est choisie dans le groupe consistant en IL-1, IL-2, IL-4, IL-7, IL-12, IFNs, GM-CSF ou TNF-alpha.

3. Utilisation d'une nanoparticule de chitosane/ARNsi, dans laquelle le chitosane possède une masse moléculaire supérieure à 10 kDa, pour la préparation d'un médicament destiné à être administré par voie intrapéritonéale.


### SEQUENCE LISTING

<110> University of Aarhus
   Howard, Ken Alan
   Deleuran, Bent
   Besenbacher, Flemming
   Kjems, Jorgen
<120> Nanoparticle-mediated treatment for inflammatory diseases
<130> 42398PC01
<160> 6
<170> PatentIn version 3.5
<210> 1
   <211> 25
   <212> RNA
   <213> Homo Sapiens
<400> 1
   gucucagccu cuucucauuc cugcu 25
<210> 2
   <211> 26
   <212> RNA
   <213> Homo Sapiens
<400> 2
   uacagagucg ggaagaguaa ggacga 26
<210> 3
   <211> 25
   <212> RNA
   <213> Homo Sapiens
<400> 3
   cuuccucucu uucucucccu uguga 25
<210> 4
   <211> 27
   <212> RNA
   <213> Homo Sapiens
<400> 4
   aggaaggaga gaaagagagg gaacacu 27
<210> 5
   <211> 27
   <212> DNA
   <213> Homo Sapiens
<400> 5
   tccuuccucu cuuucucucc cuuguga 27
<210> 6
   <211> 27
   <212> DNA
   <213> Homo Sapiens
<400> 6
   tcacaaggga gagaaagaga ggaagga 27